(19) Europäisches Patentamt
European Patent Office
Office européen des brevets

(11) Veröffentlichungsnummer: **0 172 519**
**B1**

# (12) EUROPÄISCHE PATENTSCHRIFT

(45) Veröffentlichungstag der Patentschrift: **23.11.88**

(51) Int. Cl.⁴: **C 07 C 175/00**

(21) Anmeldenummer: **85110094.1**

(22) Anmeldetag: **12.08.85**

## (54) Verfahren zur Herstellung von Jononen.

(30) Priorität: **24.08.84 DE 3431131**

(43) Veröffentlichungstag der Anmeldung: **26.02.86 Patentblatt 86/9**

(45) Bekanntmachung des Hinweises auf die Patenterteilung: **23.11.88 Patentblatt 88/47**

(84) Benannte Vertragsstaaten: **CH DE FR GB LI NL**

(56) Entgegenhaltungen: **DE-A-1 668 505**
**DE-A-1 917 132**

(73) Patentinhaber: **BASF Aktiengesellschaft, Carl-Bosch- Strasse 38, D-6700 Ludwigshafen (DE)**

(72) Erfinder: **Hoffmann, Werner, Dr., 14 Horatio Street Apt. 11 D, New York, N.Y. 10014 (US)**
Erfinder: **Janitschke, Lothar, Dr., Wormser Gasse, D-6711 Kleinniedesheim (DE)**
Erfinder: **Arnold, Lothar, Dr., Hausackerweg 11, D-6900 Heidelberg (DE)**

EP 0 172 519 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft ein verbessertes Verfahren zur kontinuierlichen Herstellung von α- und/oder β-Jonon durch Cyclisieren von Pseudojonon mittels konzentrierter Schwefelsäure in Gegenwart von organischen Lösungs- oder Verdünnungsmitteln und durch Verdünnen des Reaktionsgemisches mit Wasser.

Es ist bekannt, daß bei der Cyclisierung von Pseudojonon in Gegenwart von Säuren wie Schwefelsäure oder Phosphorsäure ein Gemisch von α- und β-Jonon erhalten wird. Das Verhältnis der Mengen, in welchen diese Verbindungen entstehen, ist stark von den Bedingungen, unter denen die Reaktion stattfindet, abhängig.

Da sowohl α-Jonon als auch β-Jonon von großer technischer Bedeutung sind, hat es nicht an Versuchen gefehlt, ein möglichst vorteilhaftes Verfahren zu deren Herstellung zu entwickeln.

Besonders bewährt haben sich Verfahren zur Cyclisierung von Pseudojonon mit konzentrierter Schwefelsäure. Da diese Umsetzung stark exotherm verläuft, ist es sehr wichtig, die entstehende Reaktionswärme möglichst schnell abzuführen, um örtliche Überhitzungen zu vermeiden. Zu diesem Zweck wurden dem Reaktionsgemisch gemäß den bekannten Verfahren Verdünnungsmittel zugegeben. So werden beispielsweise gemäß den DE-PS-1 080 105 und 1 668 505 aliphatische oder cycloaliphatische Kohlenwasserstoffe verwendet. Nachteilig an diesem Verfahren ist, daß sich in den Reaktionsgefäßen relativ schnell Harze abscheiden, die den kontinuierlichen Betrieb stören.

Gemäß der IN-PS-77 225 wird die Umsetzung in Gegenwart von aliphatischen Chlorkohlenwasserstoffen wie Methylenchlorid, Ethylendichlorid, Chloroform und Tetrachlorkohlenstoff bei Temperaturen von -10°C bis +10°C durchgeführt.

Gemäß der Beschreibung der DE-OS-1 568 108 ist dieses indische Verfahren nachteilig, da die aliphatischen Chlorkohlenwasserstoffe mit Schwefelsäure Chlorwasserstoff abspalten, wodurch die verwendeten Apparate in kurzer Zeit korrodieren. Zur Vermeidung dieser Nachteile wird empfohlen, die Cyclisierung bei -25 bis +10°C in einem Gemisch aus niedrig-siedenden Kohlenwasserstoffen und Chlorkohlenwasserstoffen durchzuführen. Nachteilig an beiden letztgenannten Verfahren ist, daß man die Reaktionstemperatur mit aufwendigen Kühlmitteln niedrig halten muß, um gute Jonon-Ausbeuten zu erzielen.

Weiterhin sind Verfahren bekannt, bei denen die beträchtliche Cyclisierungswärme durch Siedekühlung mit Flüssiggasen abgeführt wird. So arbeitet man gemäß dem Verfahren der DE-PS-1 668 496 mit flüssigem Schwefeldioxid, dem Verfahren der DE-PS-1 668 505 mit Propan, Butan oder Isobutan und dem Verfahren der DE-PS-1 917 132 mit Methylchlorid und bei Temperaturen von -25°C bis Raumtemperatur, vorzugsweise Temperaturen unterhalb von +10°C.

Die gemäß diesen Verfahren erzielten Ergebnisse sind im allgemeinen recht gut. Nachteilig hieran ist der große Aufwand, der nötig ist, um das bei der Reaktion verdampfte Gas wieder zu verflüssigen. Beim Arbeiten in flüssigem Schwefeldioxid weist das Reaktionsprodukt außerdem einen intensiven Geruch nach Schwefeldioxid auf.

Aus der CS-PS-179 046, der SU-PS-458 540 und der SU-PS-547 445 sind fern er Verfahren zur Herstellung von β-Jonon bekannt, bei denen eine gute Durchmischung der Reaktanten und eine schnelle Wärmeabfuhr dadurch erreicht werden, daß man einen Dünnfilmreaktor verwendet. Nachteilig an den beiden letztgenannten Verfahren ist, daß man pro $m^2$ Dünnfilmfläche und Stunde nur etwa 3 bis 6 kg β-Jonon erhält, und daß somit eine Übertragung in den industriellen Maßstab zu riesigen Apparaturen führen würde. Nachteilig an dem Verfahren des tschechischen Patents ist, daß man zur Erzielung guter Ausbeuten bei Temperaturen zwischen 10 und 15°C arbeiten muß, wodurch wiederum teure Kühlmittel notwendig werden.

Bei allen bekannten Verfahren bildet sich immer ein Gemisch von α- und ß-Jonon. Nach den DE-PS-1 080 105, 1 668 496 und 1 668 505 erhält man bei Reaktionstemperaturen von -20 bis 0°C bevorzugt β-Jonon, während bei Temperaturen von -10 bis 25°C der α-Jonon-Gehalt ansteigt. β-Jonon ist insbesondere ein wichtiges Vorprodukt für die technische Herstellung von Vitamin A. Ein hoher Gehalt an α-Jonon, führt dabei zu einer Verminderung der Ausbeute. α-Jonon dagegen ist ein begehrter Riechstoff, welcher in reiner Form oder im Gemisch mit dem ebenfalls Riechstoffcharakter besitzenden β-Jonon gehandelt wird. Hierbei steigt mit steigendem Gehalt an α-Jonon die Feinheit des Duftes und damit der Preis des Gemisches stark an.

Es war daher die Aufgabe der Erfindung, ein Verfahren zu entwickeln, mit dem sowohl möglichst hochprozentiges α-Jonon als auch möglichst reines ß-Jonon auf möglichst vorteilhafte Weise in hohen Ausbeuten und Raum-Zeit-Ausbeuten hergestellt werden können.

Es wurde nun ein sehr vorteilhaftes Verfahren zur kontinuierlichen Herstellung von Jononen der allgemeinen Formeln I und II

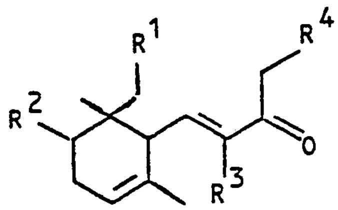

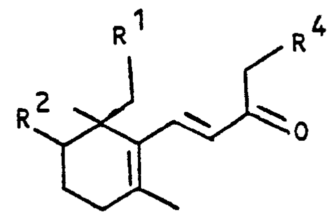

(I; α-Isomeres) (II; β-Isomeres),

in denen $R^1$ bis $R^4$ für H, $-CH_3$ oder $-C_2H_5$ stehen, durch Cyclisieren von Pseudojononen der allgemeinen Formel III

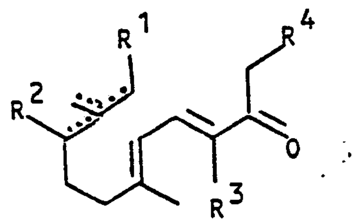

(III),

in der jeweils eine der gestrichelten Linien für eine weitere C-C-Bindung steht, mittels konzentrierter Schwefelsäure in Gegenwart von organischen Lösungs- oder Verdünnungsmitteln unter Kühlung und durch anschließendes Verdünnen des Reaktionsgemisches mit Wasser gefunden, das dadurch gekennzeichnet ist, daß man die Umsetzung in aliphatischen Ketonen mit 5 bis 10 Kohlenstoffatomen als Lösungs- bzw. Verdünnungsmittel durchführt.

Besonders vorteilhaft gestaltet sich das Verfahren, wenn man die Umsetzung in Methylisopropylketon oder von Diisopropylketon durchführt.

Bei der erfindungsgemäßen Cyclisierung ergeben sich die im folgenden aufgeführten Vorteile gegenüber der Cyclisierung gemäß den bekannten Verfahren.

1. Man arbeitet in homogener Phase, wodurch Harzabscheidungen vermieden werden und ein guter Wärmeaustausch gewährleistet ist.
2. Gegenüber dem Arbeiten in heterogener Phase, wie sie bei der Cyclisierung in Gegenwart von Kohlenwasserstoffen und/oder Halogenkohlenwasserstoffen auftritt, erzielt man um 2 bis 5 % höhere Ausbeuten.
3. Je nach Wahl der Parameter kann man Jonongemische mit 1 bis ca. 50 % α-Jonon bzw. ca. 50 bis ca. 90 % β-Jonon aus Pseudojonon herstellen.
4. Nach dem Verdünnen des Reaktionsgemisches mit Wasser dienen die Ketone gleich als Extraktionsmittel.

Als Pseudojonone der Formel (III) sind insbesondere folgende Verbindungen geeignet: das eigentliche Pseudojonon (6,10-Dimethyl-undeca-3,5,9-trien-2-on) und sein sogenanntes α-Isomeres, das 6,10-Dimethyl-undeca-3,5,10-trien-2-on sowie Pseudoiron (6,9,10-Trimethyl-undeca-3,5,9-trien-2-on), die Methyl-pseudojonone (3,6,10-Trimethyl-undeca-3,5,9-trien-2-on, 7,11-Dimethyl-dodeca-4,6,10-trien-3-on und 6,10-Dimethyl-dodeca-3,5,9-trien-2-on) und 8,12-Dimethyl-trideca-5,7,11-trien-4-on.

Die Konzentration der bei der Cyclisierung verwendeten Schwefelsäure kann zwischen 50 und 100-Gew.-% liegen. Vorzugsweise verwendet man handelsübliche 95 bis 98 %-ige Schwefelsäure. Pro Mol Pseudojonon der Formel (III) verwendet man im allgemeinen 2 bis 15 mol, vorzugsweise 2 bis 7 mol Schwefelsäure. Bei Verwendung von etwa 2 bis 3 mol Schwefelsäure pro Mol III erhält man überwiegend α-Jonon, während man beim Einsatz von mehr als 5 mol Schwefelsäure pro mol III β-Jonon mit einem Gehalt von weniger als 2 % α-Jonon erhält.

Als Ketone mit 5 bis 10 Kohlenstoffatomen kommen im wesentlichen Diethylketon, Methyl-n-propylketon, Methylisopropylketon, Diisopropylketon, Di-n-propylketon, Ethylisopropylketon, Methylisobutylketon, Diisobutylketon, Methylisoamylketon, vorzugsweise Methylisopropylketon und Diisopropylketon in Betracht. Sie werden im allgemeinen in Mengen von 0,1 bis etwa 10 mol pro mol III eingesetzt. Größere Mengen sind zwar möglich, bringen aber keine technischen Vorteile mehr.

Die Reaktionstemperaturen können - 20 bis etwa +150°C insbesondere - 10 bis +60°C betragen. Die Verweilzeit kann je nach Reaktionstemperatur 0,1 Sekunden bis etwa 1 Stunde betragen.

Das Verfahren kann diskontinuierlich durchgeführt werden, jedoch ist eine kontinuierliche Verfahrensweise im allgemeinen vorteilhafter. Zur kontinuierlichen Durchführung eignet sich jeder Mischraum, in welchem eine sehr intensive Durchmischung und eine sehr gute Wärmeabführung gewährleistet sind.

Je nach den angewendeten Reaktionsbedingungen erhält man bei dem erfindungsgemäßen Verfahren

Gemische mit unterschiedlichem Gehalt an α- bzw. β-Isomeren. Allgemein lassen sich folgende Gesetzmäßigkeiten feststellen:

Gemische mit hohen α-Jonon-Gehalten erhält man bei kurzen Verweilzeiten, tiefen Temperaturen, niedriger Schwefelsäure- und hoher Keton-Konzentration. Entsprechend erhält man Gemische mit hohen β-Jonon-Gehalten bei längeren Verweilzeiten, höheren Temperaturen, hoher Schwefelsäure- und niedriger Ketonkonzentration.

In den folgenden Beispielen 1 bis 10 werden der Einfluß von Reaktionstemperatur und Reaktionszeit auf die Ausbeuten an α- bzw. β-Jonon aufgezeigt.

**Beispiele 1 bis 12**

In einem 1 1-Rührkolben wurden 300 g konzentrierte (98 %-ige) Schwefelsäure vorgelegt. Unter intensivem Rühren (6 bis 800 U/min) wurde unter Außenkühlung mit einem Methanol/Trockeneisbad so rasch wie möglich die Lösung von 100 g 6, 10-Dimethyl-undeca-3,5,9-trien-2-on (91,65 %-ig entsprechend 0,48 mol; bestimmt auf einer 2 m Carbowax-Säule, bei 200°C isotherm, GC-Gerät: Hewlett Packard) in 100 g Diisopropylketon zugegeben. Die Zulaufgeschwindigkeit wurde der jeweiligen Versuchstemperatur angepaßt, d.h. war davon abhängig, wie schnell die Reaktionswärme über die Kühlung abgeführt werden konnte. Die notwendige Zulaufzeit ist in der folgenden Tabelle angegeben. Nach Zulaufende wurde die jeweils aus der Tabelle ersichtliche Nachreaktionszeit eingehalten. Anschließend wurde das Reaktionsgemisch auf 500 g Eis gegossen, die sich abscheidende organische Phase abgetrennt, mit Natriumbicarbonatlösung (ca. 10 ml) neutral gewaschen und das Diisopropylketon bei 70°C und 300 bis 20 mbar abdestilliert. Das zurückbleibende Jonongemisch wurde dann bei 0,1 mbar und einer Badtemperatur von maximal 160°C über eine Destillationsbrücke destilliert und der β- bzw. α-Jonon-Gehalt gaschromatographisch bestimmt (2 m Carbowax-Säule, 200°C isotherm, 0,5 µl, Empfindlichkeit 6). In der nachfolgenden Tabelle sind die Reaktionsbedingungen und die Ergebnisse dargestellt. Die Ausbeuteangaben sind bezogen auf umgesetztes Ausgangsmaterial.

**Tabelle 1**

| Nr. | Versuchs-temp. [°C] | Zulaufzeit [min] | Nachreak-tionszeit [min] | Destillat: [g] | α-Jonon-gehalt*) [%] | β-Jonon-gehalt*) [%] | Rück-stand [g] | Um-satz [%] | Ausbeute α-Jonon [%] | Ausbeute β-Jonon [%] |
|---|---|---|---|---|---|---|---|---|---|---|
| 1 | -10 | 10 | 15 | 84 | 20,8 | 74 | 8 | 100 | 19 | 67,8 |
| 2 | 0 | 4 | 15 | 88 | 5,3 | 84,2 | 9 | 99 | 5,1 | 80,8 |
| 3 | +10 | 4,5 | 15 | 86 | 2,7 | 91,9 | 11 | 100 | 2,5 | 86,2 |
| 4 | +20 | 3 | 15 | 84 | 3,9 | 91 | 14 | 100 | 3,5 | 83,4 |
| 5 | +30 | 2,3 | 15 | 76 | 3,9 | 84,1 | 23 | 100 | 3,2 | 69,7 |
| 6 | -10 | 10 | 1 | 84 | 30,3 | 63,1 | 9 | 98 | 27,8 | 57,8 |
| 7 | 0 | 5 | 1 | 87 | 19,8 | 75,3 | 9 | 100 | 18,8 | 71,5 |
| 8 | +10 | 4 | 1 | 86 | 13,1 | 82,6 | 10 | 100 | 12,3 | 77,5 |
| 9 | +20 | 3 | 1 | 84 | 5,6 | 90,6 | 13 | 100 | 5,1 | 83,0 |
| 10 | +30 | 2,3 | 1 | 83 | 3,3 | 90,6 | 13 | 100 | 3,0 | 82,1 |
| 11 | +10 | 4,5 | 15 | 88 | 2,0 | 92,0 | 10 | 100 | 1,9 | 88,3 |
| 12 | +10 | 4,5 | 15 | 85 | 2,0 | 94,5 | 13 | 100 | 1,8 | 87,7 |

*) des Destillats

**Beispiel 13**

Man arbeitete wie in Beispiel 3 beschrieben, verwendete jedoch anstelle von 100 g Diisopropylketon 100 g Diethylketon. Man erhielt β-Jonon in eimer Ausbeute von 85,8 % und α-Jonon in einer Ausbeute von 2,9 % der Theorie.

**Beispiel 14**

Man arbeitete wie in Beispiel 3 beschrieben, verwendete jedoch anstelle von 100 g Diisopropylketon 100 g Methyl-isopropylketom. Dabei wurdem Ausbeuten von 85,7 % an β-Jonon und 3,5 % an α-Jonon ethalten.

**Beispiele 15 bis 19**

Analog den Beispielen 1 bis 12 wurden 100,2 g 6,10-Dimethyl-undeca-3,5,9-trien-2-on (96 %-ig entsprechend 0,5 mol; bestimmt wie in Beispielen 1 bis 12) mit jeweils 300 g konz. $H_2SO_4$ und im den aus der Tabelle ersichtlichen Mengen an Diisopropylketon-umgesetzt. Die Versuchsbedingungen und Ergebnisse sind in Tabelle 2 zusammengefaßt. Die Ausbeuteangaben sind bezogen auf umgesetztes Ausgangsprodukt.

Beispiele 15 und 16 bzw. 17, 18 und 19 zeigen, daß die α-Jononausbeuten bei gleichbleibender Temperatur mit der Menge des Lösungsmittels steigen, die β-Jononausbeuten dagegen fallen. Die Beispiele 17 bis 19 zeigen, daß die β-Jononausbeuten bei höheren Temperaturen höher sind als bei den Temperaturen gemäß den Beispielen 15 und 16. Dies ist im Gegensatz zu den Angaben gemäß dem Stand der Technik.

**Tabelle 2**

Zusammensetzung des Destillats

| Nr. | Versuchs-temperatur [°C] | Nachreak-tionszeit [min] | Menge Diiso-propylketon [ml] | Destillat [g] | Ausgangs-material [%] | α-Jonon-gehalt [%] | β-Jonon-gehalt [%] |
|---|---|---|---|---|---|---|---|
| 15 | (-10) - (-15) | 15 | 100 | 87,5 | - | 17,88 | 75,50 |
| 16 | (-10) - (-15) | 15 | 200 | 89,9 | 6,43 | 55,68 | 26,07 |
| 17 | 10 - 20 | 15 | 100 | 87,4 | 16,32 | 1,93 | 64,76 |
| 18 | 10 - 25 | 15 | 200 | 85,5 | 2,76 | 14,78 | 75,63 |
| 19 | 10 - 20 | 15 | 300 | 89,4 | 1,94 | 30,32 | 52,85 |

| Nr. | Rück-stand [g] | Umsatz [%] | Ausbeute α-Jonon [%] | Ausbeute β-Jonon [%] | Ausbeute α- + β-Jonon [%] |
|---|---|---|---|---|---|
| 15 | 6,1 | 100 | 16,27 | 68,71 | 84,98 |
| 16 | 7,6 | 93,98 | 55,39 | 25,94 | 81,33 |
| 17 | 8,1 | 85,17 | 12,84 | 69,12 | 81,97 |
| 18 | 9,0 | 97,55 | 13,47 | 68,94 | 82,41 |
| 19 | 11,9 | 98,20 | 28,77 | 50,04 | 78,75 |

**Beispiele 20 bis 23**

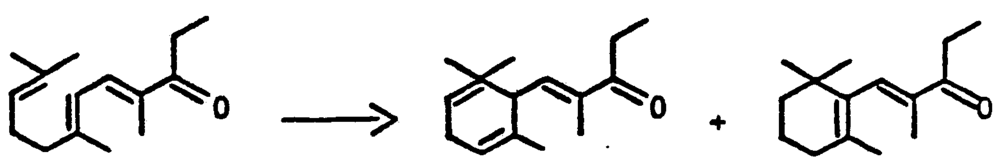

α β

Analog den Beispielen 1 bis 12 wurden jeweils 120,8 g 4,7,11-Trimethyl-dodeca-4,6,10-trien-3-on (Dimethylpseudojonon; 93,5 %-ig entsprechend 0,5 mol) mit jeweils 300 g konz. $H_2SO_4$ und den aus Tabelle 3 ersichtlichen Mengen Diisopropylketon umgesetzt. Die Versuchsbedingungen und Ergebnisse sind in Tabelle 3 zusammengefaßt. Die Ausbeuteangaben sind bezogen auf umgesetzte Ausgangsverbindung.

Ein Vergleich der Beispiele 20 und 21 zeigt, wie die Ausbeuten an α-Jonon mit Erhöhung der Reaktionstemperaturen drastisch steigen, die Beispiele 21 bis 23 den Einfluß der Ketonmenge auf die Ausbeuten an α-Jonon bzw. β-Jonon.

**Tabelle 3**

Zusammensetzung des Destillats

| Nr. | Versuchs-temperatur [°C] | Nachreak-tionszeit [min] | Menge Diiso-propylketon [ml] | Destillat [g] | Ausgangs-material [%] | Dimethyl-α-Jonon-gehalt [%] | Dimethyl-β-Jonon-gehalt [%] |
|---|---|---|---|---|---|---|---|
| 20 | (-10) - (-15) | 15 | 100 | 86,2 | 1,13 | 53,82 | 35,71 |
| 21 | 10 - 20 | 15 | 100 | 78,1 | 1,39 | - | 88,66 |
| 22 | 10 - 20 | 15 | 200 | 80,1 | 2,30 | - | 91,03 |
| 23 | 10 - 20 | 15 | 300 | 86,4 | 1,40 | 44,32 | 38,81 |

| Nr. | Rück-stand [g] | Umsatz [%] | Ausbeute Dimethyl-α-Jonon [%] | Ausbeute Dimethyl-β-Jonon [%] | Ausbeute Dimethyl-α- + β-Jonon [%] |
|---|---|---|---|---|---|
| 20 | 24,9 | 99,12 | 42,48 | 28,19 | 70,67 |
| 21 | 28,1 | 99,01 | - | 63,48 | 63,48 |
| 22 | 28,5 | 98,33 | - | 67,30 | 67,3 |
| 23 | 32,4 | 98,90 | 35,14 | 30,77 | 65,9 |

**Beispiele 24 bis 28**

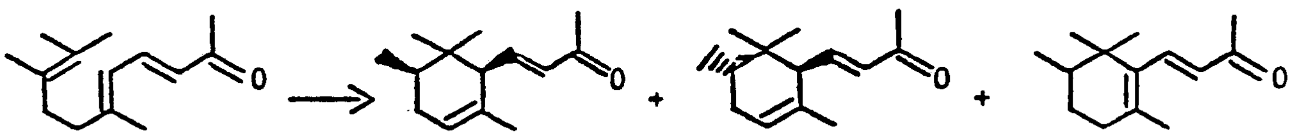

cis-α trans-α ß

Analog den Beispielen 1 bis 12 wurden 108 g 6,9,10-Trimethyl-undeca-3,5,9-trien-2-on (Pseudoiron; 96,5 %-ig entsprechend 0,5 mol) mit jeweils 300 g konz. $H_2SO_4$ und variierenden Mengen Diisopropylketon umgesetzt. Die Versuchsbedingungen und Ergebnisse sind in Tabelle 4 zusammengefaßt. Die Ausbeuteangaben sind bezogen auf umgesetzte Ausgangsverbindung.

**Tabelle 4**

Zusammensetzung des Destillats

| Nr. | Versuchs-temperatur [°C] | Nachreak-tionszeit [min] | Menge Diiso-propylketon [ml] | Destillat [g] | Ausgangs-material [%] | cis-α-Iron-gehalt [%] |
|---|---|---|---|---|---|---|
| 24 | (-10) - (-15) | 15 | 100 | 92,1 | - | - |
| 25 | (-10) - (-15) | 15 | 200 | 92,5 | - | 11,56 |
| 26 | 10 - 20 | 15 | 100 | 91,0 | - | 16,88 |
| 27 | 10 - 20 | 15 | 200 | 93,1 | - | - |
| 28 | 10 - 20 | 15 | 300 | 85,8 | - | 2,56 |

| Nr. | trans-α-Iron-gehalt [%] | β-Iron-gehalt [%] | Rück-stand [g] | Umsatz [%] | Ausbeute α-Iron [%] | Ausbeute β-Iron [%] | Ausbeute α- + β-Iron [%] |
|---|---|---|---|---|---|---|---|
| 24 | 51,73 | 44,67 | 10,3 | 100 | 46,20 | 39,89 | 86,08 |
| 25 | 49,38 | 26,18 | 11,2 | 100 | 54,66 | 23,48 | 78,19 |
| 26 | 16,88 | 74,81 | 12,1 | 100 | 29,78 | 66,01 | 95,79 |
| 27 | 34,63 | 46,42 | 14,7 | 100 | 31,26 | 41,91 | 73,17 |
| 28 | 41,47 | 41,16 | 17,8 | 100 | 36,63 | 34,24 | 70,87 |

**Beispiele 29 bis 33**

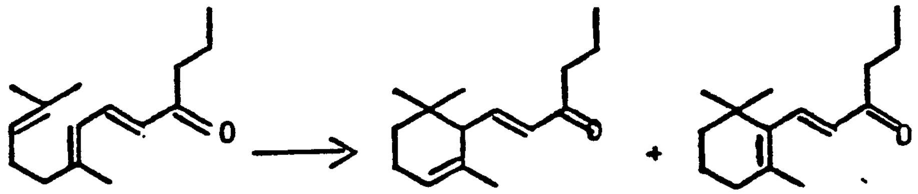

α β

Analog zu den Beispielen 1 bis 10 wurden 128 g 8,12-Dimethyl-trideca-5,7,11-trien-4-on (Ethylpseudojonon; 86 %-ig entsprechend 0,5 mol) mit jeweils 300 g konz. $H_2SO_4$ und in den aus Tabelle 5 ersichtlichen Mengen an Diisopropylketon ungesetzt.

Die Aufarbeitung erfolgte analog Beispielen 1 bis 10. Die Reaktionsbedingungen und Ergebnisse sind in Tabelle 5 zusammengefaßt. Die Ausbeuteangaben sind bezogen auf umgesetzte Ausgangsverbindung.

Ein Vergleich der Ergebnisse aus Beispielen 29 und 30 sowie 31, 32 und 33 zeigt deutlich den Einfluß der Ketonmenge auf die Ausbeuten an α- und β-Jonon. Ein Vergleich der Ergebnisse aus den Beispielen 29 und 31 zeigt deütlich den Einfluß der Versuchstemperatur auf die Ausbeuten an α- und β-Jonon.

**Tabelle 5**

Zusammensetzung des Destillats

| Nr. | Versuchs-temperatur [°C] | Nachreak-tionszeit [min] | Menge Diiso-propylketon [ml] | Destillat [g] | Ausgangs-material [%] | Ethyl-α-Jonon-gehalt [%] | Ethyl-β-Jonon-gehalt [%] |
|---|---|---|---|---|---|---|---|
| 29 | (-10) - (-15) | 15 | 100 | 110,9 | 9,07 | 16,88 | 64,16 |
| 30 | (-10) - (-15) | 15 | 200 | 118,6 | 36,71 | 34,81 | 12,98 |
| 31 | 10 - 20 | 15 | 100 | 109,5 | 1,64 | 5,34 | 80,78 |
| 32 | 10 - 20 | 15 | 200 | 112,4 | 2,10 | 2,87 | 74,72 |
| 33 | 10 - 20 | 15 | 300 | 113,8 | 0,54 | 34,96 | 40,23 |

| Nr. | Rück-stand [g] | Umsatz [%] | Ausbeute Ethyl-α-Jonon [%] | Ausbeute Ethyl-β-Jonon [%] | Ausbeute Ethyl-α- + β-Jonon [%] |
|---|---|---|---|---|---|
| 29 | 10,8 | 90,87 | 18,70 | 71,07 | 89,76 |
| 30 | 11,1 | 60,48 | 61,95 | 23,10 | 85,05 |
| 31 | 17,9 | 98,37 | 5,40 | 81,61 | 87,01 |
| 32 | 24,9 | 97,87 | 2,99 | 77,89 | 80,89 |
| 33 | 13,3 | 99,44 | 36,31 | 41,78 | 78,10 |

**Beispiele 34 bis 37**

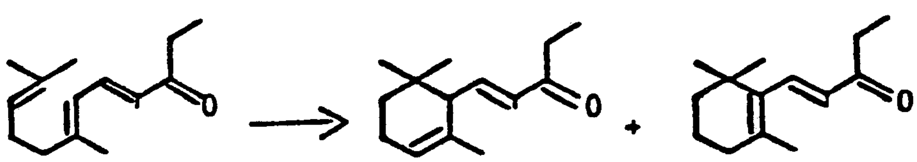

α β

Analog den Beispielen 1 bis 10 wurden 109 g 7,11-Dimethyl-dodeca-4,6,10-trien-3-on (Methylpseudojonon; 94,6 %-ig entsprechend 0,5 mol) mit jeweils 300 g konz. $H_2SO_4$ und in den aus Tabelle 6 ersichtlichen Mengen an Diisopropylketon umgesetzt.

Die Aufarbeitung erfolgte analog den Beispielen 1 bis 10. Die Reaktionsbedingungen und Ergebnisse sind in Tabelle 6 zusammengefaßt.

**Tabelle 6**

Zusammensetzung des Destillats

| Nr. | Versuchstemperatur [°C] | Nachreaktionszeit [min] | Menge Diisopropylketon [ml] | Destillat [g] | Ausgangsmaterial [%] | Methyl-α-Jonongehalt [%] | Methyl-β-Jonongehalt [%] |
|---|---|---|---|---|---|---|---|
| 34 | (-10) - (-15) | 15 | 100 | 90,5 | - | 15,20 | 55,65 |
| 35 | (-10) - (-15) | 15 | 200 | 83,2 | 11,21 | 45,64 | 28,88 |
| 36 | (-10) - (-15) | 15 | 300 | 86,2 | 41,16 | 32,59 | 14,16 |
| 37 | 10 - 20 | 15 | 200 | 78,4 | - | 7,68 | 77,55 |

| Nr. | Rückstand [g] | Umsatz [%] | Ausbeute Methyl-α-Jonon [%] | Ausbeute Methyl-β-Jonon [%] | Ausbeute Methyl-α- + β-Jonon [%] |
|---|---|---|---|---|---|
| 34 | 10,3 | 100 | 13,34 | 48,83 | 62,17 |
| 35 | 20,5 | 90,96 | 40,48 | 25,62 | 66,10 |
| 36 | 23,8 | 65,61 | 41,52 | 18,05 | 59,57 |
| 37 | 22,1 | 100 | 5,84 | 58,95 | 64,79 |

**Patentansprüche**

1. Verfahren zur Herstellung von Jononen der allgemeinen Formeln I und II

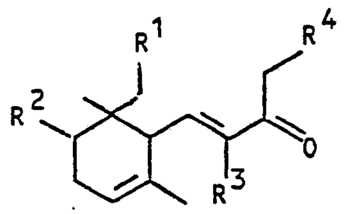

(I; α-Isomeres)

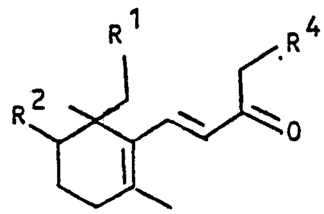

(II; β-Isomeres),

in denen $R^1$ bis $R^4$ für H, $-CH_3$ oder $-C_2H_5$ stehen, durch Cyclisieren von Pseudojononen der allgemeinen Formel III

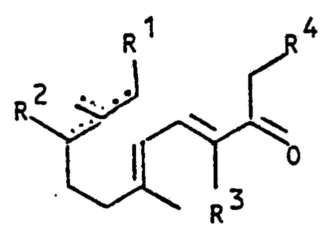

(III),

in der jeweils eine der gestrichelten Linien für eine weitere C-C-Bindung steht, mittels konzentrierter Schwefelsäure in Gegenwart von organischen Lösungs- oder Verdünnungsmitteln unter Kühlung und durch anschließendes Verdünnen des Reaktionsgemisches mit Wasser, dadurch gekennzeichnet, daß man die Umsetzung in aliphatischen Ketonen mit 5 bis 10 Kohlenstoffatomen als Lösungs- bzw. Verdünnungsmittel durchführt.

2. Verfahren gemäß Anspruch 1, dadurch gekennzeichnet, daß man die Umsetzung in Methylisopropylketon oder Di-isopropylketon durchführt.

**Claims**

1. A process for the preparation of ionones of the formulae I and II

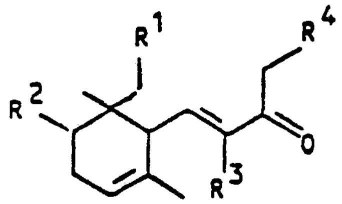

(I; α-isomer)

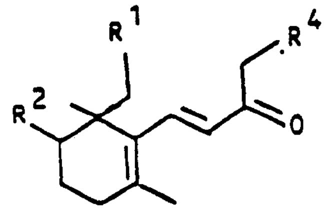

(II; β-isomer)

where $R^1$, $R^2$, $R^3$ and $R^4$ are H, $-CH_3$ or $-C_2H_5$, by cyclizing pseudoionones of the formula III

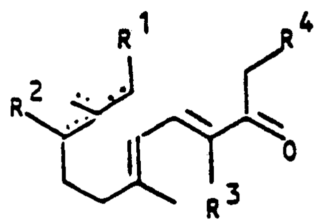

(III)

where one of the broken lines represents a further C-C bond, by means of concentrated sulfuric acid in the presence of an organic solvent or diluent, with cooling, and subsequently diluting the reaction mixture with water, wherein the reaction is carried out in an aliphatic ketone of 5 to 10 carbon atoms as the solvent or diluent.

2. A process as claimed in claim 1, wherein the reaction is carried out in methyl isopropyl ketone or diisopropyl ketone.

**Revendications**

1. Procédé de préparation de ionones de formules générales I et II

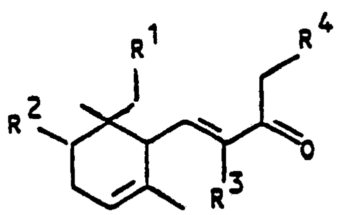

(I; isomère α)

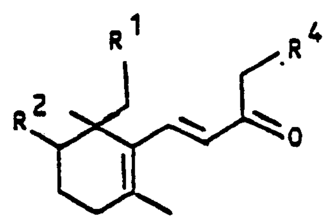

(II; isomère β)

dans lesquelles $R^1$ à $R^4$ sont mis pour H, $-CH_3$ ou $C_2H_5$, par cyclisation de pseudo-ionones de formule générale III,

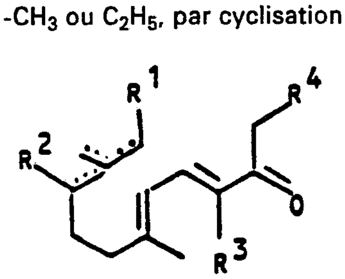

(III)

dans laquelle chacune des lignes pointillées est mise pour une autre liaison C-C, à l'aide d'acide sulfurique concentré en présence de solvants ou diluants organiques, sous refroidissement, et par dilution ultérieure du mélange réactionnel avec de l'eau, caractérisé en ce qu'on effectue la réaction dans des cétones aliphatiques ayant de 5 à 10 atomes de carbone en tant que solvant ou diluant.

2. Procédé selon la revendication 1, caractérisé en ce qu'on effectue la réaction dans la méthylisopropylcétone ou la diisopropylcétone.